# EUROPEAN PATENT SPECIFICATION

(11) **EP 1 851 013 B1**
(45) Date of publication and mention of the grant of the patent: **11.01.2012**
(21) Application number: 06709781.6
(22) Date of filing: 16.02.2006
(51) Int. Cl.: B26B 13/06, B26B 13/20, A61B 17/32

(54) **IMPROVEMENTS IN OR RELATING TO SCISSORS**
VERBESSERUNGEN AN ODER IM ZUSAMMENHANG MIT EINER SCHERE
AMÉLIORATIONS À DES CISEAUX OU LES CONCERNANT

(30) Priority: 16.02.2005 GB 0503210
(43) Date of publication of application: 07.11.2007
(73) Proprietor: George, Samuel, Weybridge, Surrey KT13 9SB (GB)
(72) Inventor: George, Samuel, Weybridge, Surrey KT13 9SB (GB)
(74) Representative: Cummings, Sean Patrick
(86) International application number: PCT/GB2006/000544
(87) International publication number: WO 2006/087554

(56) References cited:
- GB-A- 2 345 857
- US-A- 2 127 190
- US-A- 3 846 910
- US-A- 5 007 170
- US-A1- 2004 015 181

## Description

The present invention relates to scissors and more particularly but not exclusively to surgical scissors.

Surgical scissors are indispensable tools for surgeons, operating theatre staff and other medical practitioners and are used for cutting and excising patient tissue or sutures. These scissors need to be able to cut cleanly, effectively and accurately, to be robust, and to be suitable for sterilisation. It can be appreciated that accuracy of cutting is important in any surgical procedure. Achieving a clean cut is particularly important for minimising trauma and inflammation to the surrounding tissues.

Conventional surgical scissors comprise two scissor members, each scissor member having a distal blade portion and a proximal handle portion. The blade portion has a cutting edge, a contacting face, an outer face and a distal tip. A finger opening is at the proximal end of the handle portion. The scissor members are pivotally connected to each other at a pivot axis between the blade portion and the handle portion such that their contacting faces can mutually abut when closed. As with conventional scissors, as the distal tips of the blade portions are moved closer together by squeezing together the handle portions, the contacting faces of the blade portions slide relative to each other parallel to their planes. In so doing, the respective cutting edges mutually intersect and contact each other at a point of contact which travels distally along the cutting edges as the blade members come together. Cutting is achieved by the shearing action of the opposed cutting edges of the blade portions at the point of contact. Conventionally, surgical scissors are ergonomically adapted for either left-handed or right-handed use.

This conventional scissor design is ineffective when cutting tough fibrous tissue such as ligaments, or large sections of tissue. In those situations, the blade members of the scissors tend to splay apart or the tissue slips away, thus achieving either an imprecise cut or no cut at all. In addition, the misalignment caused by the blade members splaying apart can result in tissue becoming trapped between the splayed blade members rendering the scissors useless until the trapped tissue is freed. In order to minimise the chances of blade splaying, the surgeon must perform a number of smaller cuts on tough tissue as opposed to one clean cut. However, a series of small cuts, results in an imprecise, untidy and ragged cut; it also prolongs the procedure.

Often, surgical scissors are unable to withstand the rigours of surgery and sterilisation. They may need to be repaired frequently or discarded and replaced. Commonly, the two blades are held against each other by a pivot pin which loosens and increases the risk of the blade splaying apart in use. Also the blades tend to become blunt on recurrent use and hence require frequent sharpening.

A possible way of overcoming some disadvantages of conventional surgical scissors would be to use stiffer blades so that they do not splay apart as readily. Stiffer blades may be achieved by using a stiffer material, which would be more expensive, or by making the blades thicker. However, thicker blades are both undesirable and impractical as the scissors may be too bulky to manoeuvre in confined spaces, heavier and therefore more tiring to use, and more expensive to make.

An object of the present invention is to provide improved scissors which are robust, cut cleanly and effectively and avoid or minimise the disadvantages of current scissors as outlined above. A scissors according to the preamble of claim 1 is also known from US 3,846,910 The invention resides in scissors having first and second pivotably connected elongate blade members, each blade member having a handle portion and a blade portion, wherein at least one of the handle portions comprises a wedge means co-operable with the other handle portion during a cutting stroke to urge the handle portions apart laterally while biasing the blade portions laterally together to oppose relative lateral movement of the blade portions away from each other during the cutting stroke, characterised in that the wedge means has a thick end and a thin end, and an area of contact between the wedge means and the other handle portion moves from the thin end towards the thick end during the cutting stroke to provide a continuous wedging force throughout the cutting stroke.

Also, described herein are scissors having first and second pivotably connected elongate blade members wherein the second blade member is associated with a restraining member extending at least partially along said blade member and spaced therefrom to define a slot or channel in which the first blade member is at least partially received during cutting to restrain relative lateral movement of the blade members away from each other during a cutting stroke.

The restraining member resists splaying deflection of the blade members, which facilitates cutting of tough tissues such as ligaments.

There may be a clearance between the first blade member and the restraining member when the scissors are closed at the end of a cutting stroke, with the first blade member being narrower than the width of the slot or channel. In this way, the restraining member is not necessarily in contact with the first blade member during normal cutting but as soon as minor deflection of the first blade member takes up the clearance, the restraining member begins to limit further deflection.

It is also possible for the restraining member to bear against the first blade member during the cutting stroke. For example, the restraining member may bear resiliently against the first blade member to deflect resiliently as the first blade member moves during the cutting stroke. In this way, the restraining member can act continually to resist deflection of the first blade member. Indeed, the first blade member may be thicker or wider than the width of the slot or channel, so that resilient deflection of the restraining member is essential to accommodate the first blade member. In order to facilitate cleaning and sterilisation of the scissors, the restraining member may be circular in cross-section.

The second blade member and the restraining member may be conjoined along at least part of their shared length. For example, the second blade member and the restraining member may together define a U-shaped cross-section. This is stiff and easy to fabricate: for example, the second blade member and the restraining member may be formed integrally with one another. Alternatively, the second blade member and the restraining member may be separate along most of their shared length, for example being joined at opposed ends of the restraining member. This means that the second blade member and the restraining member can together define an open-bottomed slot, which is easier to clean and less likely to give rise to jamming.

Alternatively, the second blade member and the restraining member can be joined at a proximal end of the restraining member such that a distal end of the restraining member is not joined to the second blade member. Advantageously, the proximal end of the restraining member can form a fulcrum between the first blade member and the second blade member.

In conventional manner, the first blade member defines a first cutting edge and the second blade member defines a second cutting edge whose point of mutual intersection moves distally during the cutting stroke. The restraining member can define a third cutting edge, the first cutting edge passing between the second and third cutting edges during the cutting stroke. At least one of these cutting edges may be chamfered or bevelled laterally, to prevent tissues moving out of the blades: they may also or alternatively be serrated, curved, or scalloped longitudinally to prevent tissues moving out of the blades.

Advantageously, the point or area of contact between the first blade portion and the restraining member can move in register with the point of intersection of the opposing cutting edges, to resist splaying deflection of the first blade portion opposite the point at which the deflecting force is exerted.

An edge of the restraining member is preferably parallel to the cutting edge of the second blade portion, and more preferably level or co-planar with the cutting edge of the second blade portion.

For optimum cutting efficiency, the restraining member is preferably shorter than the second blade portion defining a difference in height at least equal to the width of the slot or channel between them.

Beneficially, the restraining member and the first blade member have opposed co-operable faces and the arrangement is preferably such that an increasing proportion of the first blade member is received in the slot or channel as the cutting stroke progresses. This lends progressively greater support to the distal end of the first blade member.

In order that this invention may be more readily understood, currently preferred embodiments will now be further described by way of example with reference to the accompanying drawings, in which Figures 1 to 14 do not form part of the invention:
Figure 1 is a perspective view of one embodiment of a pair of surgical scissors;
Figure 2 is a perspective view of a second embodiment of a pair of surgical scissors;
Figure 3 is a perspective view of a third embodiment of a pair of surgical scissors;
Figure 4 is a perspective view of a fourth embodiment of a pair of surgical scissors;
Figure 5 is a perspective view of a fifth embodiment of a pair of surgical scissors;
Figure 6 is a perspective view of a sixth embodiment of a pair of surgical scissors;
Figure 7 is a perspective view of a seventh embodiment of a pair of surgical scissors;
Figures 8(a) to 8(c) are cross-sections of any of the embodiments of Figures 1 to 7; and
Figures 9(a) to 9(c) are cross-sections of any of the embodiments of Figures 1 to 7.
Figure 10 is a perspective view of a variant of any of the embodiments of Figures 1 to 7;
Figure 11 is a perspective view of a further variant of any of the embodiments of Figures 1 to 7;
Figure 12 is a perspective view of an eighth embodiment of a pair of surgical scissors;
Figure 13 is a perspective view of an eighth embodiment of a pair of surgical scissors;
Figure 14 is a perspective view of an eighth embodiment of a pair of surgical scissors;
Figure 15 is a perspective view of a ninth embodiment of a pair of surgical scissors according to the invention; and
Figure 16 is a perspective view of a tenth embodiment of a pair of surgical scissors according to the invention.

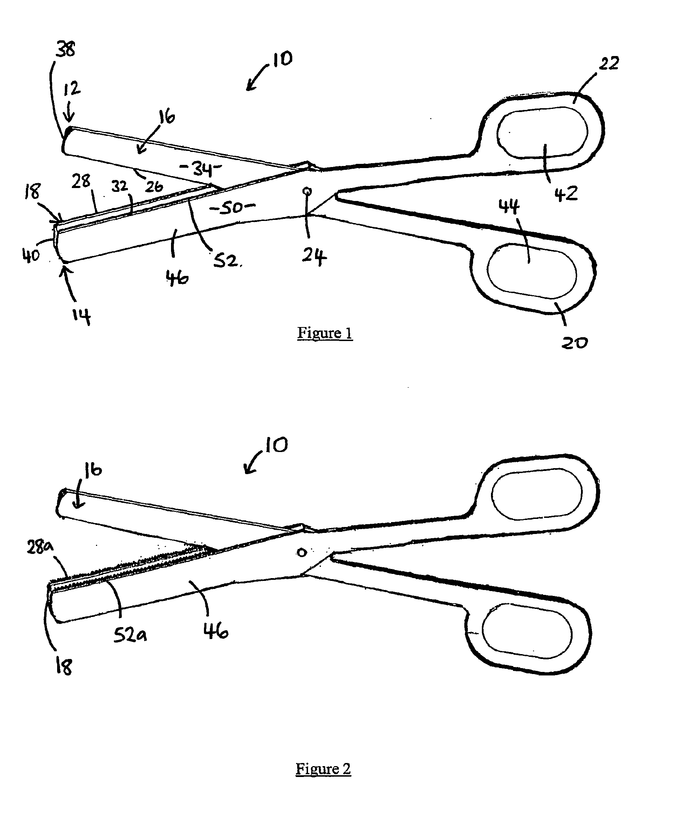

Referring firstly to Figure 1, which is not of the present invention, there is shown a pair of surgical scissors 10 having first and second elongate scissor members 12, 14. In conventional manner, each scissor member 12, 14 has a distal blade portion 16, 18 and a proximal handle portion 20, 22 and the scissor members 12, 14 are pivotally connected to each other by a rivet, screw, stud or pin 24 at a pivot axis between the blade portion 16, 18 and the handle portion 20, 22. Also conventionally, the blade portion 16, 18 has a cutting edge 26, 28, a contacting face 30, 32, an outer face 34, 36 and a distal tip 38, 40, and a finger opening 42, 44 is provided at the proximal end of the handle portion 20, 22.

Unconventionally, a restraining member 46 is associated with the second blade portion 18 and may be integral with, or distinct from but attached to, the second blade portion 18. An integral restraining member 46 is shown in Figure 1. The restraining member 46 is of substantially the same shape as the second blade portion 18 and has an inner face 48, an outer face 50 and a flat top edge 52 orthogonal to the inner and outer faces 48, 50.

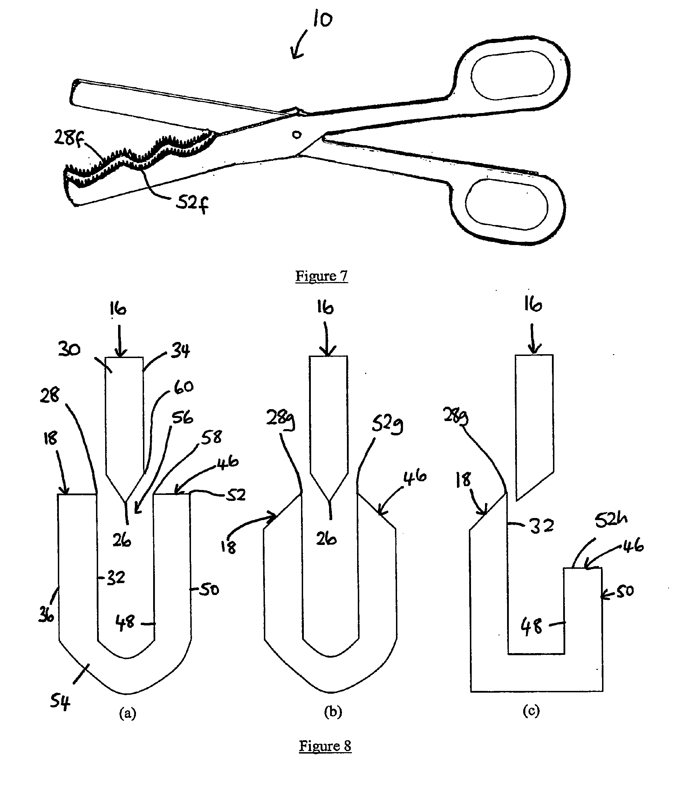

The inner and outer faces 48, 50 of the restraining member 46 lie parallel to the contacting face 32 and outer face 36 of the second blade portion 18, with the inner face 48 of the restraining member 46 opposed to and spaced from the contacting face 32 of the second blade portion 18. The integral restraining member 46 and second blade portion 18 of Figure 1 are defined by a U-shaped cross-section (as shown in Figure 8(a)) wherein the second blade portion 18 is one arm of the U and the restraining member 46 is the other arm of the U, both arms being joined by a common base 54.

The contacting face 32 of the second blade portion 18 and the inner face 48 of the restraining member 46 define a channel or slot 56 for receiving the cutting edge portion of the first blade portion 16 during cutting. When the scissors 10 are closed, a substantial part of the first blade portion 16 including its cutting edge 26 lies in the channel or slot 56, sandwiched between the second blade portion 18 and the restraining member 46. The outer face 34 of the first blade portion 16 and the inner face 48 of the restraining member 46 may be spaced from one another to define a small clearance, or they may abut one another in a sliding fit. The restraining member 46 may deflect resiliently to facilitate sliding movement of the first blade portion 16 if needs be. This will be necessary if the channel or slot 56 is narrower than the thickness of the first blade portion 16 to be received in it.

In conventional manner, the cutting action involves squeezing together the handle portions 20, 22 which pivots the distal tips 38, 40 of the blade portions 16, 18 towards each other, from an open to a closed position. During this movement, the opposing cutting edges 26, 28 of the first and second blade portions 16, 18 slide relative to each other in mutually intersecting manner. The point of intersection and hence the point of contact between the opposing cutting edges 26, 28 is initially near the pivot 24 and moves distally along the cutting edges 26, 28 as the distal tips 38, 40 of the blade portions 16, 18 swing closer together.

The restraining member 46 restrains lateral splaying movement of the first blade portion 16 away from the second blade portion 18. Such lateral movement would be transverse to the length of the blade portions 16, 18 and parallel to the pivot axis defined by the pivotable connection 24. As the distal tips 38, 40 of the first and second blade portions 16, 18 swing closer together, an increasing proportion of the first blade portion 16 is received into the slot or channel 56 defined by the contacting face 32 of the second blade portion 18 and the inner face 48 of the restraining member 46. The first blade portion 16 is thereby prevented from splaying away from the second blade portion 18 by virtue of contact with the restraining member 46.

If there is a clearance between the first blade portion 16 and the restraining member 46, lateral movement of the first blade portion 16 is resisted as soon as the first blade portion 16 takes up this clearance and its outer face 34 comes into contact with the inner face 48 of the restraining member 46. If there is no clearance, that is if the outer face 34 of the first blade portion 16 is in sliding contact with the inner face 48 of the restraining member 18 lateral movement of the first blade portion 16 is resisted from the outset. In either case, the point or area of contact between the first blade portion 16 and the restraining member 46 moves along in register with the point of intersection of the opposing cutting edges 26, 28, so that splaying deflection of the first blade portion 16 is resisted opposite the point at which the deflecting force is exerted. Advantageously, this enables cutting of tough or slippery items which might otherwise cause the blades to splay away from each other.

The top edge 52 of the restraining member 46 may provide a third cutting edge 58 which is optionally parallel to the cutting edge 28 of the second blade portion 18 as shown. This third cutting edge 58 may be co-operable with a secondary cutting edge 60 on the first blade portion 16, or may be co-operable with the same cutting edge 26 on the first blade portion 16 that co-operates with the cutting edge 28 of the second blade portion 18. The second and third cutting edges 60, 58 in Figure 1, also shown in Figure 8(a), are flat and co-planar, lying in a plane orthogonal to the plane in which the first blade portion 16 moves about the pivotable connection between the scissor members 12, 14.

The embodiments of Figures 2 to 7, which are not of the present invention, differ from Figure 1 in that the cutting edges of the first and second blade portions and the top edge of the restraining member have been adapted to improve the grip on tissue by the scissors before and during cutting.

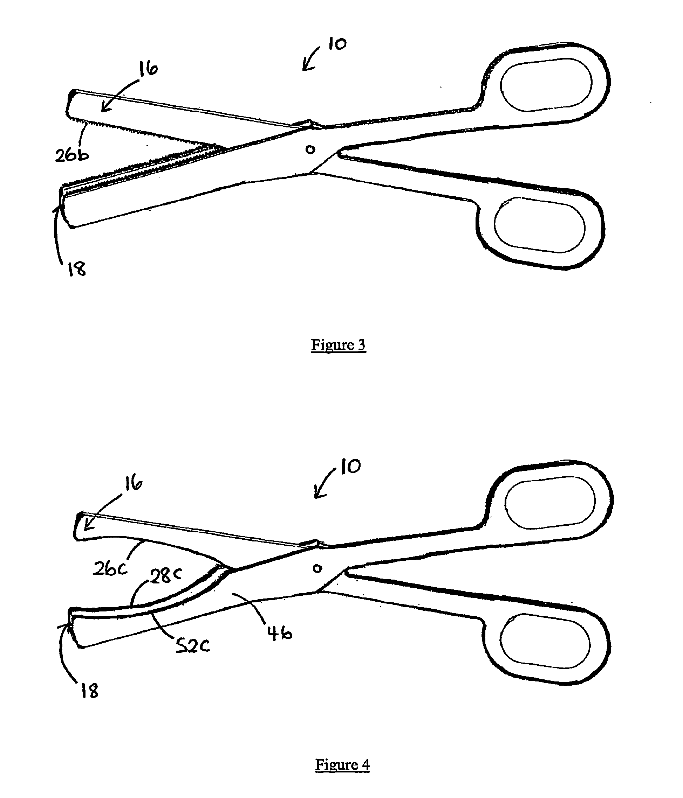

In the embodiment of Figure 2, the cutting edge 28a of the second blade portion 18 and the top edge 52a of the restraining member 46 are serrated. The embodiment of Figure 3 differs from that of Figure 2 only in that the cutting edge 26b of the first blade portion 16 is also serrated.

In the embodiment of Figure 4, the cutting edges 26c, 28c of the first and second blade portions 16, 18 and the top edge 52c of the restraining member 46 are concave-curved. Although not shown, it is feasible that only one cutting edge might be curved in this manner: the other cutting edge(s) may be straight or otherwise shaped. Alternatively, the edges opposite the cutting edges of the distal blade portions 16, 18 and/or the proximal handle portions 20, 22 may also be concave-curved to reduce the overall weight of the scissors and improve manageability.

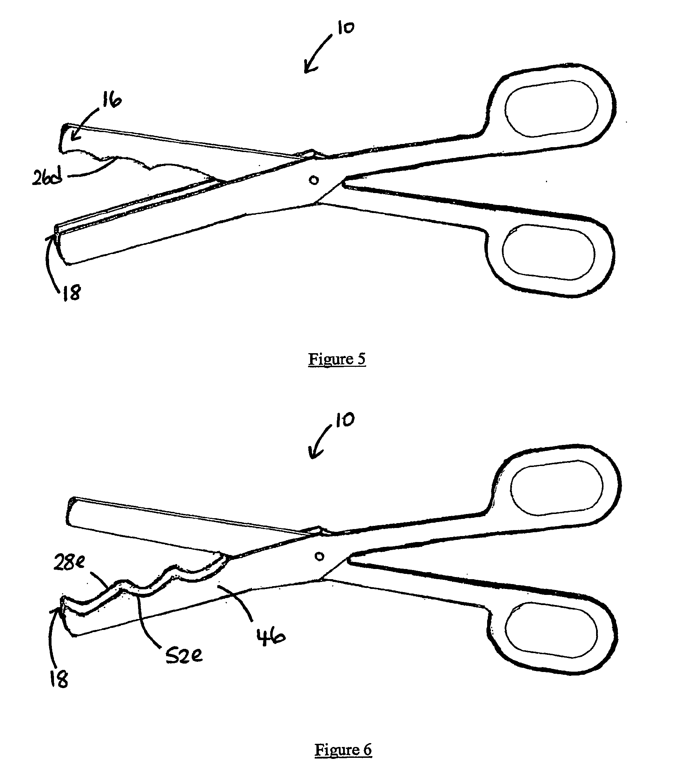

In the embodiment of Figure 5, the cutting edge 26d of the first blade portion 16 has a series of concave curvatures, or peaks and troughs, defining a wavy scalloped line. Conversely in the embodiment of Figure 6, only the cutting edge 28e of the second blade portion 18 and the top edge 52e of the restraining member 46 have a series of concave curvatures. However, it will be apparent that all of the cutting edges could have similar formations.

Figure 7 shows a further embodiment which differs from that of Figure 6 in that the concave curvatures of the top edge 52f of the restraining member 46 and the cutting edge 28f of the second blade portion 18 are serrated. Alternatively, just one of the scalloped edges 28f, 52f may be serrated. Although not shown, it will be apparent that the curved edges of the embodiments shown in Figures 4 and 5 may also be serrated. Further alternative embodiments are possible where the scissors comprise at least one flat serrated edge in combination with the serrated and/or non-serrated curved edges described above. Serrated edges are advantageous in that they have an improved grip on the tissues and require sharpening less often than edges without serrations.

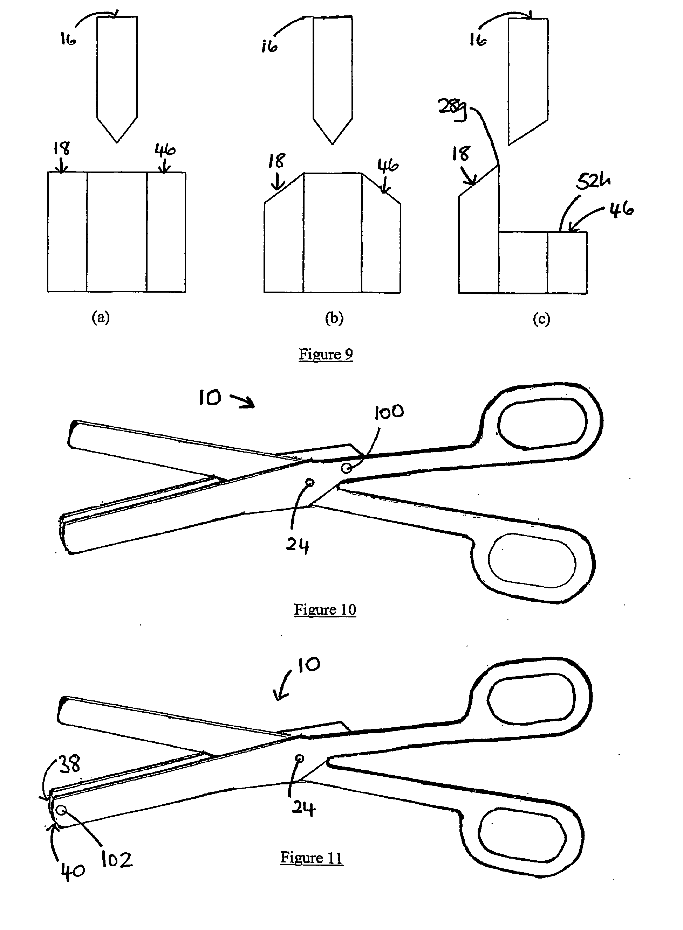

Figures 8(b) and 8(c) and Figures 9(a) to 9(c), which are not of the present invention, show cross-sectional views of different variants of the scissors of all preceding embodiments. Figures 8(b) and 8(c) differ from Figures 1 to 7 and 8(a) in that the cutting edge 28g of the second blade portion 18 is chamfered instead of flat. In Figure 8(b), the top edge 52g of the restraining member 46 is also chamfered. In Figure 8(c) only the cutting edge 28g of the second blade portion 18 is chamfered whilst the top face 52h of the restraining member 46 is flat and orthogonal to its the inner and outer faces 48, 50. In this variant, the restraining member 46 is shorter than the second blade portion 18, their difference in height being equal to the width of the gap between them i.e. the distance between the opposed contacting face 32 of the second blade portion 18 and the inner face 48 of the restraining member 46. The shortened restraining member 46 allows easier access to the channel 56 between the restraining member 46 and the second blade portion 18, which is advantageous for cleaning and sterilising purposes. It will be appreciated that the ratio of the height difference between the restraining member 46 and the second blade portion 18 to the width of the gap between them may be more or less than 1:1 i.e. the restraining member may be shorter or longer than the width of the gap between the restraining member and the second blade portion.

The variants in Figures 9(a) to 9(c), 10 and 11 differ from all of the preceding embodiments in that the second blade portion 18 and the restraining member 46 are not a U shape in cross-section. Otherwise, they correspond to Figures 8(a) to 8(c) respectively: for example Figure 9(c) shows a chamfered cutting edge 28g of the second blade portion 18 and a flat top edge 52h of the restraining member 46 orthogonal to its the inner and outer faces 48, 50, the difference in height between the restraining member 46 and the second blade portion 18 being equal to the width of the gap between them. Whilst they may still be integral, the second blade portion 18 and the restraining member 46 do not have a common base extending along their length but instead may be joined at their ends. Alternatively, the second blade portion 18 and the restraining member 46 may have a common base extending part of the way along their length or along parts of their length. Put another way, the second blade portion 18 and the restraining member 46 may be bridged at some points along their length by a common base which may be integral with either, or both, the second blade portion 18 and the restraining member 46, or distinct but attached to the second blade portion 18 and the restraining member 46. Although not shown, the length of the restraining member may be shorter than the length of the second blade portion. This is particularly beneficial in sharp pointed scissors where a shorter length restraining member prevents the twisting or the crossing-over of the sharp pointed distal tips of the first and second blade portions.

Figure 10, which is not of the present invention, shows the second blade portion 18 joined to the restraining member 46 by a rivet, screw, stud or pin 100 at their proximal ends, and located close to the pivot axis 24 between the blade portion 16, 18 and the handle portion 20, 22. The embodiment shown in Figure 11 differs from that shown in Figure 10 in that the second blade portion 18 and the restraining member 46 are joined by a rivet, screw, stud or pin 102 at their distal tips 38, 40 instead of their proximal ends. Although not shown, it will be appreciated that the second blade portion 18 and the restraining member 46 can be joined by a rivet at both their distal and their proximal tips.

The variants of Figures 8(b) and (c), 9, 10 and 11 can be applied to all the preceding embodiments, for example the embodiment of Figure 2 with the cross-sectional appearance of Figure 9(a).

A benefit of the variants in Figures 9(a) to 9(c) and the variants described in the preceding paragraph is that there is less scope for dirt or other detritus to get stuck in the channel or slot between the second blade portion and the restraining member. The fully or partially open bottom allows the slot 56 to be flushed out more readily upon being cleaned or sterilised. The movement of the first blade portion into the slot also lends a self cleaning action in use.

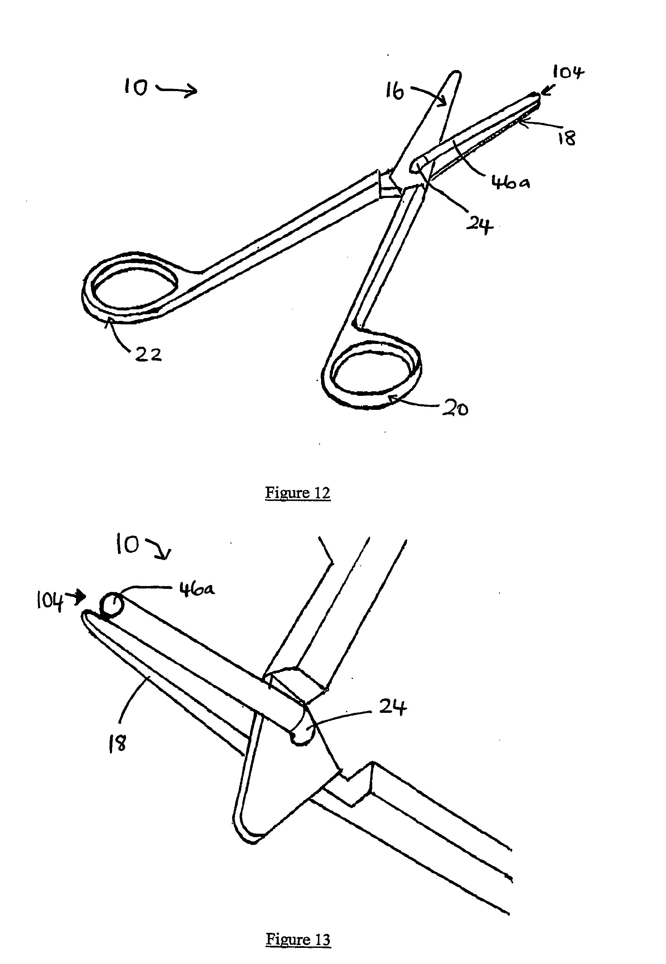

A further alternative embodiment, which is not of the present invention, is shown in Figures 12 to 14, which differs from the previous embodiments in that it has a restraining member 46a which is substantially the same length as the second blade portion 18, extending along the length of the second blade portion 18, but is cylindrical in shape, having a substantially circular transverse cross-section. The restraining member 46a is a piece of wire or rod and has a free or unattached distal end or tip 104. The other end of the restraining member 46a forms the fulcrum at the pivot axis between the blade portions 16, 18 and the handle portions 20, 22. Thus that end of the restraining member 46a extends through a hole in the first blade portion 16 and is fixed to the second blade portion 18. Advantageously, the rounded profile of the restraining member 46a is easy to clean using normal cleaning and sterilising techniques. Although not shown, the restraining member 46a may be attached to or integral with the second blade portion 18 at both ends of the restraining member which would reduce splaying of the first blade portion 16.

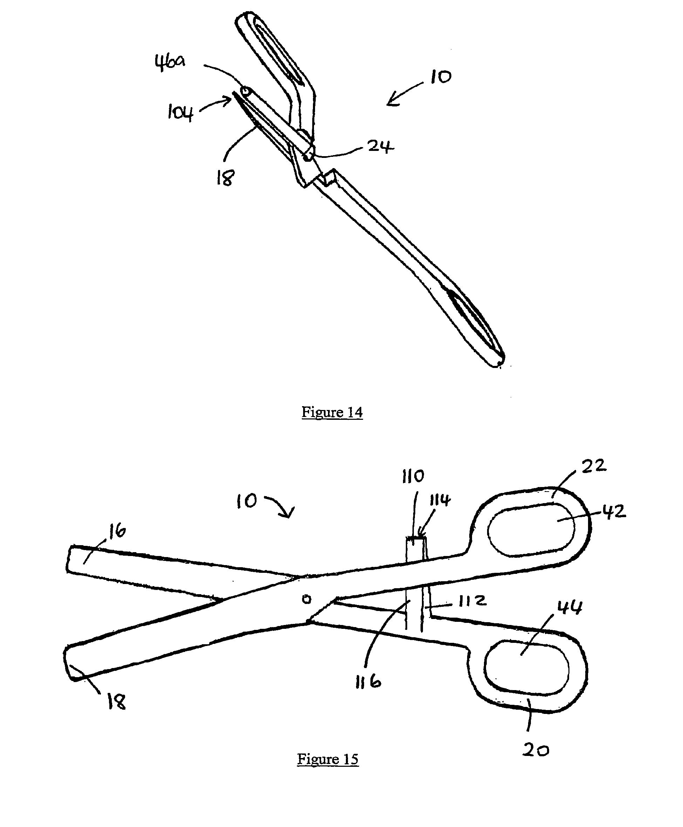

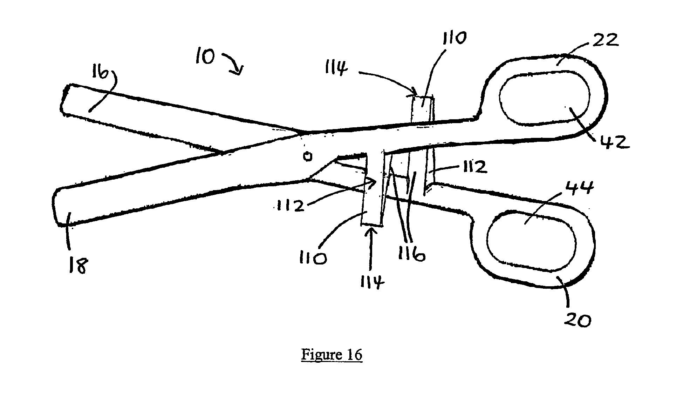

Figures 15 and 16 show an embodiment of the scissors of the present invention which differs from the preceding embodiments in that instead of having a restraining member associated with the second blade portion 18, at least one of the proximal handle portions of the scissors 10 comprise at least one protuberance or projection in the shape of a wedge extending in a direction transverse to the length of the handle portion, towards the other proximal handle portion. The wedge projection 110 has a thick end 112, a thin end 114, and at least one inclined face 116 which is in sliding contact with the other blade member. The wedge projection 110 is substantially triangular in cross-section.

Figure 15 shows a pair of scissors 10 comprising such a wedge projection 110 extending from the first handle portion 20 towards the second handle portion 22. The wedge projection 110 is located approximately midway along the length of the first handle portion 20. The thick end 112 of the wedge projection 110 may be integral with, or be distinct from but attached to, the first handle portion 20, in a fixed position with respect to the first handle portion 20. The wedge projection 110 may also be arranged so that it is moveable along the first handle portion 20. Optionally, the scissors 10 having the moveable wedge projection 110 may comprise a fixing mechanism to allow the moveable wedge projection to be fixed or secured in position along the first handle portion 20.

In use, as the handle portions 20, 22 are brought closer together in conventional scissoring action, the inclined face 116 of the wedge projection 110 and the opposing face (not shown) of the second handle portion 22 slide relative to each other. The area of contact between them on the inclined face 116 of the wedge projection 110 moves from its thin end 114 towards its thick end 112, resulting in the first and second handle portions 20, 22 being splayed or forced apart laterally. This has the effect of forcing or biasing the blade portions 16, 18 towards each other in a lateral direction i.e. transversely to the movement of the blade portions 16, 18 in normal scissoring motion during cutting. This biasing of the blade portions 16, 18 towards each other opposes lateral splaying movement of the blade portions 16, 18 during cutting. The positioning of the wedge projection 110 and the angle of its inclined face 116 is such that the wedge projection 110 does not impart any lateral force between the handle portions 20, 22 when the scissors 10 (handle portions and blade portions) are in an open position.

The wedge face 116 is continuous such that the wedging force increases, or at least does not decrease, during and throughout the cutting stroke for effective cutting. This is distinguished from known ratchet mechanisms on handle portions which can be said to comprise a series of wedges with discontinuous wedge faces which do not provide a continuous wedging force during a cutting stroke.

Figure 16 differs from the embodiment shown in Figure 15 in that the second handle portion 22 also comprises a wedge projection 110 extending towards the first handle portion 20. This second wedge projection 110 is similar to that of Figure 15 both in appearance and functionality. The inclined face 116 of the second wedge projection 110 is in sliding contact with the opposing face of the first handle portion 20. Although not shown, the handle portions 20, 22 may comprise more than one wedge projection which may be positioned at any point along their length. These wedge projections 110 may be fixed or moveable with respect to the handle portions 20, 22.

The advantage of having one or more moveable wedge projections 110 is that the wedging effect, and hence the force that splays the handle portions 20, 22 apart and the blade portions 16, 18 together, can be adjusted for reliable cutting while minimising the effort required of the user.

The scissors described herein may be embodied in other specific forms without departing from the essential attributes. For example, the outer faces of the second blade portion and the restraining member do not have to be parallel. It is also possible for the first blade member to have an associated restraining member, defining a channel or slot that receives and minimises deflection of the second blade member. However, it will be apparent that the restraining member may also have the effect of stiffening the blade member with which it is associated. The restraining member need not be of substantially the same shape or length as the second blade portion. Although the scissors of the present invention have been described as surgical scissors, they may also be equally suitable for non-surgical and non-medical applications such as cutting textiles and other materials.

## Claims

1. Scissors (10) having first and second pivotably connected elongate blade members, each blade member having a handle portion (20, 22) and a blade portion (16, 18), wherein at least one of the handle portions (20) comprises a wedge means (110) co-operable with the other handle portion (22) during a cutting stroke to urge the handle portions (20, 22) apart laterally while biasing the blade portions (16, 18) laterally together to oppose relative lateral movement of the blade portions (16, 18) away from each other during the cutting stroke, **characterised in that** the wedge means (110) has a thick end (112) and a thin end (114), and an area of contact between the wedge means (110) and the other handle portion (22) moves from the thin end (114) towards the thick end (112) during the cutting stroke to provide a continuous wedging force throughout the cutting stroke.

2. The scissors (10) of Claim 1, wherein the wedge means (110) has an inclined face (116) which is in sliding contact with the other handle portion (22) during the cutting stroke.

3. The scissors (10) of Claim 1 or Claim 2, wherein the wedge means (110) protrude from one handle portion (20) towards the other handle portion (22).

4. The scissors (10) of any preceding Claim, wherein the wedge means (110) is located approximately midway along the length of the handle portion (20).

5. The scissors (10) of any preceding Claim, further comprising a second wedge means (110) associated with one of the handle portions (20, 22).

6. The scissors (10) of Claim 5, wherein the second wedge means (110) is associated with the other handle portion (22).

## Patentansprüche

1. Schere (10) mit einem ersten und einem zweiten drehbar verbundenen länglichen Blattelement, von denen jedes einen Griffabschnitt (20, 22) und einen Blattabschnitt (16, 18) aufweist, wobei mindestens einer der Griffabschnitte (20) ein Keilmittel (110) umfasst, das bei einer Schneidbewegung so mit dem anderen Griffabschnitt (22) zusammenwirkt, dass die Griffabschnitte (20, 22) lateral voneinander weggedrückt werden, während die Blattabschnitte (16, 18) lateral zusammengedrückt werden und der bei der Schneidbewegung voneinander weg gerichteten relativen lateralen Bewegung der Blattabschnitte (16, 18) entgegenwirken, **dadurch gekennzeichnet, dass** das Keilmittel (110) ein dickes Ende (112) und ein dünnes Ende (114) aufweist und sich eine Kontaktfläche zwischen dem Keilmittel (110) und dem anderen Griffabschnitt (22) bei der Schneidbewegung vom dünnen Ende (114) zum dicken Ende (112) hin bewegt und während der gesamten Schneidbewegung eine kontinuierliche Verkeilkraft bereitstellt.

2. Schere (10) nach Anspruch 1, bei der das Keilmittel (110) eine schräge Fläche (116) aufweist, die sich bei der Schneidbewegung in Gleitkontakt mit dem anderen Griffabschnitt (22) befindet.

3. Schere (10) nach Anspruch 1 oder 2, bei der das Keilmittel (110) von einem Griffabschnitt (20) aus zu dem anderen Griffabschnitt (22) hinragt.

4. Schere (10) nach einem der vorhergehenden Ansprüche, bei der sich das Keilmittel (110) etwa in der Mitte der Länge des Griffabschnittes (20) befindet.

5. Schere (10) nach einem der vorhergehenden Ansprüche, die ferner ein zweites Keilmittel (110) umfasst, das zu einem der Griffabschnitte (20, 22) gehört.

6. Schere (10) nach Anspruch 5, bei der das zweite Keilmittel (110) zu dem anderen Griffabschnitt (22) gehört.

## Revendications

1. Ciseaux (10) ayant un premier et un deuxième élément lame allongé reliés de manière pivotante, chaque élément lame ayant une partie poignée (20, 22) et une partie lame (16, 18), dans lesquels au moins une des parties poignées (20) comprend un moyen coin (110) capable de coopérer avec l'autre partie poignée (22) pendant un coup de ciseaux pour écarter latéralement par poussée les parties poignées (20, 22) tout en sollicitant latéralement les parties lames (16, 18) l'une vers l'autre pour s'opposer au mouvement latéral relatif des parties lames (16, 18) les éloignant l'une de l'autre pendant le coup de ciseaux, **caractérisés en ce que** le moyen coin (110) a une extrémité épaisse (112) et une extrémité mince (114), et **en ce qu'**une zone de contact entre le moyen coin (110) et l'autre partie poignée (22) se déplace de l'extrémité mince (114) vers l'extrémité épaisse (112) pendant le coup de ciseaux pour fournir une force de calage continue pendant tout le coup de ciseaux.

2. Ciseaux (10) selon la revendication 1, dans lesquels le moyen coin (110) a une face inclinée (116) qui est en contact coulissant avec l'autre partie poignée (22) pendant le coup de ciseaux.

3. Ciseaux (10) selon la revendication 1 ou la revendication 2, dans lesquels le moyen coin (110) fait saillie d'une partie poignée (20) vers l'autre partie poignée (22).

4. Ciseaux (10) selon l'une quelconque des revendications précédentes, dans lesquels le moyen coin (110) est situé environ à mi-chemin le long de la longueur de la partie poignée (20).

5. Ciseaux (10) selon l'une quelconque des revendications précédentes, comprenant en outre un deuxième moyen coin (110) associé à une des parties poignées (20, 22).

6. Ciseaux (10) selon la revendication 5, dans lesquels le deuxième moyen coin (110) est associé à l'autre partie poignée (22).
